# EUROPEAN PATENT APPLICATION

(11) **EP 1 848 074 A1**
(43) Date of publication of application: **24.10.2007**
(21) Application number: 07106546.0
(22) Date of filing: 19.04.2007
(51) Int. Cl.: H01S 3/23, H01S 3/102, H01S 3/0941, A61B 18/20

(54) **Switchable laser device and method for operating the same**

(30) Priority: 19.04.2006 EP 06112776
(71) Applicant: Multitel ASBL, 7000 Mons (BE)
(72) Inventor: Vercambre, Clément, 59750 Feignies (FR); Hernandez, Yves, 59570 Taisnières sur Hon (FR)
(74) Representative: pronovem

(57) **Abstract**

The present invention relates to a laser device (1) comprising a double clad active element (3) for emitting light and a multi-mode pumping source (5) arranged for providing energy to said double clad active element (3), said laser device being arranged for emitting light at a first wavelength determined by an absorption spectrum of said double clad active element and whereby the laser device is further arranged for emitting light at a second wavelength determined by said multi-mode pumping source (5), said multi-mode pumping source (5) being tuneable away from said absorption spectrum.

## Description

### Field of the invention

The present invention relates to the field of laser devices comprising an active light emitting element and a tuneable pumping source for providing energy to the active element.

### State of the art

First lasers appeared in the 1970s. Since this technological breakthrough a lot of new applications and possibilities have appeared beginning by improvements in the field of medicine. Due to cost issues a lot of technological work has been performed to optimise the laser structures and to render the devices as polyvalent as possible. In the objective of reducing cost and increasing the efficiency of such devices, the fibre laser has appeared in the 1990s. This technology allows realising more reliable, compact and cost effective laser equipment. Furthermore a lot of work has been done to extend the laser performance, such that a wider range of applications can be addressed. Optical parametric oscillators, for instance, allow switching the various wavelength spectra wherein a laser device emits, thus making a single device usable for various applications, thereby reducing the investment cost for the final end-user.

Certain applications such as industrial marking and cutting, medical treatment and cutting or chemical detection require different types of lasers. Detection applications often require sources that are wavelength tuneable over at least a few nm range. Marking applications require the possibility to adapt the laser spot to the dimensions of the patterns to be written. For medical applications, such as dermatology, manufacturers propose devices comprising a set of lasers arranged for emitting at different wavelength regions in order to treat different diseases with a single device.

Document WO 95/34007-A1 discloses a laser device tuneable over a few nanometres into a given wavelength emission window. In that case, tuneability is obtained by moving a grating behind optical fibres. However, such a limited tuneable range obviously does not substantially change the device characteristics and does not yield an extended field of application.

Patent application WO 02/22033-A1 relates to a laser device able to switch between two wavelengths in a mechanical way in order to provide the user with an equipment having extended capabilities. However, the proposed solution is complex and expensive. No other features than wavelength switching are added to the laser device.

WO 02/075976-A1 teaches a thermally tuneable way to optimise the optical gain in a fibre amplifier. The invention uses this kind of thermal regulation of the pump diode. The thermal shifting of the pump is used for the stabilisation and the optimisation of the laser.

Patent document EP0917262-A discloses a multi-wavelength fibre laser for telecom applications. The pump wavelength tuneability is exploited to switch between different fibre laser structures through Wavelength Division Multiplexing (WDM) splitters and combiners. The pump wavelength is switched to correspond to a different port of the WDM splitter, but the pump radiation is always absorbed by one of the fibre laser structures. The proposed approach intends to switch directly between the fibre laser emission and the pump emission.

Patent application US2005/226286-A1 presents a classical structure of an Ytterbium fibre amplifier with a compressor set up at the output to amplify pulsed signals and to compensate for any pulse duration broadening. In this application the possibility to slightly shift the pump radiation from the peak absorption spectra is mentioned. This method is used to stabilize the fibre laser radiation and to reduce its sensitivity to temperature shifts. This feature is well known in the art. Furthermore the use of 915nm pump lasers instead of 976nm in Erbium Ytterbium fibre lasers or amplifiers is also often used for this purpose. Since the absorption peak spectrum at 915nm is broader than the peak at 976nm, any wavelength change of the pump due to temperature may leave the fibre laser operation unaffected. Then despite the fact that the 915nm wavelength is less absorbed, this wavelength can be chosen for stable fibre laser operation.

In DE10241984-A1 the impact of pump wavelength on fibre absorption is described. The possibility to tune the pump wavelength is used in order to obtain a more homogeneous absorption along the fibre and a more uniform amplification of the signals. Here again, pump wavelength shifting is used for the purpose of stabilizing or optimizing the fibre laser characteristics.

### Aims of the invention

The present invention aims to provide a laser device capable of emitting light having properties that can be switched between at least two sets of characteristics. In another aspect the invention also relates to a method for operating such a laser device.

### Summary of the invention

The present invention relates to a laser device comprising a double clad active element for emitting light and a multi-mode pumping source arranged for providing energy to said active element, whereby the laser device is arranged for emitting light at a first wavelength determined by the absorption spectrum of the double clad active element. The laser device is further arranged for emitting light at a second wavelength determined by the multi-mode pumping source that can be tuned away from said absorption spectrum. The wavelength range over which the pumping source is tuneable depends on the active element that is applied. For example, in the case of an Erbium and/or Ytterbium doped fibre as active element this range is 6 nm, preferably 10 nm.

Advantageously the tuneable multi-mode pumping source is a laser diode.

In a preferred embodiment the laser device is a double clad fibre laser or a fibre amplifier.

In an advantageous embodiment the laser device further comprises a long period grating.

In a further embodiment the laser device further comprises a pump reflector at an output of the laser device.

In another advantageous embodiment the laser device further comprises a wavelength selective or reflective element. This is preferably a dichroic mirror.

In a further embodiment the second wavelength is the wavelength generated by the multi-mode tuneable pumping source.

The invention further also relates to a medical device comprising a laser device as previously described.

In a second aspect the invention relates to a method for switching between operable states of a laser device comprising the steps of :
- providing a laser device comprising a double clad active element and a tuneable multi-mode pumping source,
- emitting with the laser device light at a first wavelength determined by an absorption spectrum of the double clad active element,
- tuning the tuneable pumping source, such that a wavelength shift to a second wavelength away from the absorption spectrum is obtained,
- emitting with the laser device light at the second wavelength.

In an alternative embodiment first light is emitted at the second wavelength, whereby subsequently the tuneable pumping source is tuned to the absorption spectrum and whereby then light is emitted at the first wavelength.

In an advantageous embodiment the step of tuning is performed by changing the temperature of the tuneable pumping source.

Preferably the laser device further comprises a wavelength dependent element, such that the step of tuning can be performed by tuning over a smaller wavelength range.

In a further aspect the invention relates to a method for operating a medical device, comprising the step of carrying out the method as previously described.

### Short description of the drawings

Fig. 1 represents a general scheme of a laser device with an active element.

Fig. 2 represents a general scheme of a fibre laser device comprising an active element and a pumping laser as tuneable pumping source.

Fig. 3 represents the switching of the output light between two sets of characteristics by adjusting the temperature of the pumping source.

Fig. 4 represents an embodiment comprising a wavelength selective or reflective element.

Fig. 5 represents two examples. In Fig.5A wavelength tuning of a fibre laser is illustrated. Fig.5B shows laser beam quality tuning of a double clad doped fibre.

Fig. 6 represents a design with an improved extinction ratio.

Fig. 7 represents a two outputs laser architecture capable of increasing the extinction ratio between the two states.

Fig. 8 represents a way to work on the active element to limit the absorption coefficient out of the absorption peak.

### Detailed description of the invention

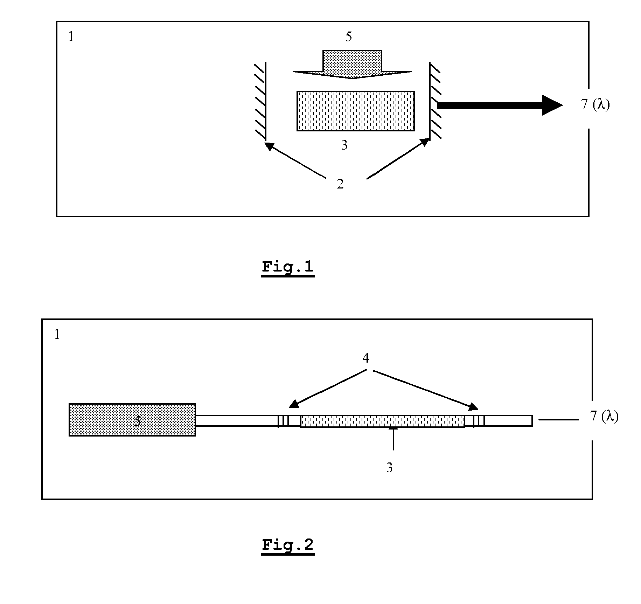

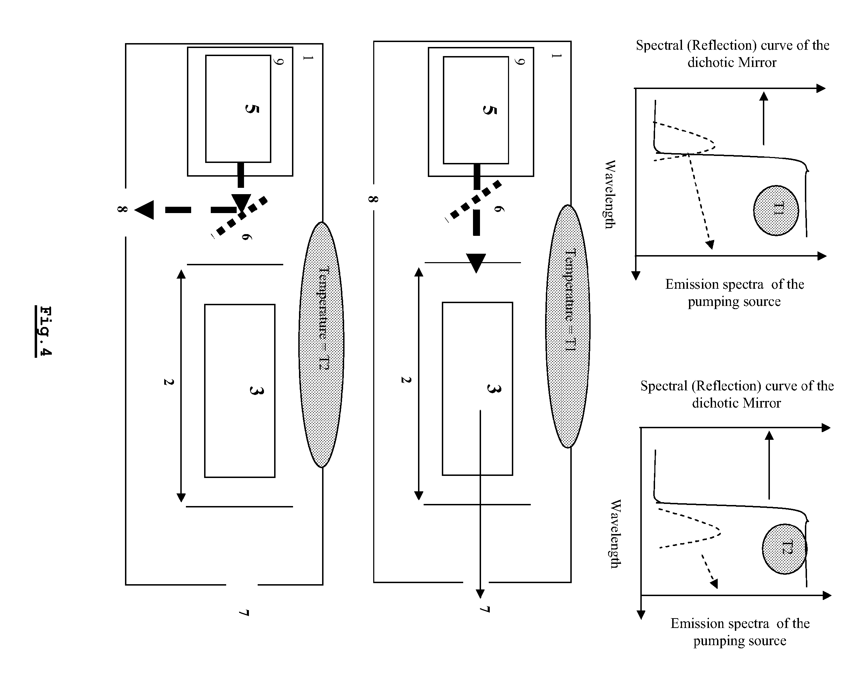

For the active element (3) in a cavity (2) of a laser device (1) to be operable, it must be supplied with energy. This energy is then converted and emitted by the active element of the device and the laser radiation appears at the output (7) (Fig.1) . In certain cases (e.g. for fibre lasers), the supplied energy can come from a tuneable pumping source (5) (Fig.2), which often is a laser diode pump. Then the active element (3) of the laser device
(1) absorbs the incident light and re-emits a laser radiation. The wavelength λ of this radiation is determined by the emission spectrum of the active element (3). Fig.4 also shows a fibre Bragg Grating based cavity (4). In many prior art disclosures measures are taken in order to keep the light of the pumping source at a wavelength corresponding to the absorption spectrum of the active element.

In order to obtain a highly versatile laser device, the present invention proposes a laser device and method for operating such device that allows switching at least between the emitted radiation of the active element (3) and the pumping radiation of the tuneable pumping source (5). Actually the output light of the laser device always derives partially from the light emitted by the pumping source and the light emitted by the active element. When the pumping source wavelength matches the absorption spectrum of the active element, the output light is predominantly derived from the active element. When the pumping source wavelength is tuned out of the absorption spectrum, then the output of the laser device is substantially derived from the pumping source. Between these two modes of operation, the ratio between the light derived from the pumping source and the light derived from the active element varies continuously with the pumping source wavelength shift. The invention then allows obtaining cost effective laser devices with more options in terms of output light properties (like wavelength and beam shape, as detailed below).

However, this is no limitation to the scope of the invention, as the concept is equally compatible with other types of lasers (solid-state, gas, dye...).

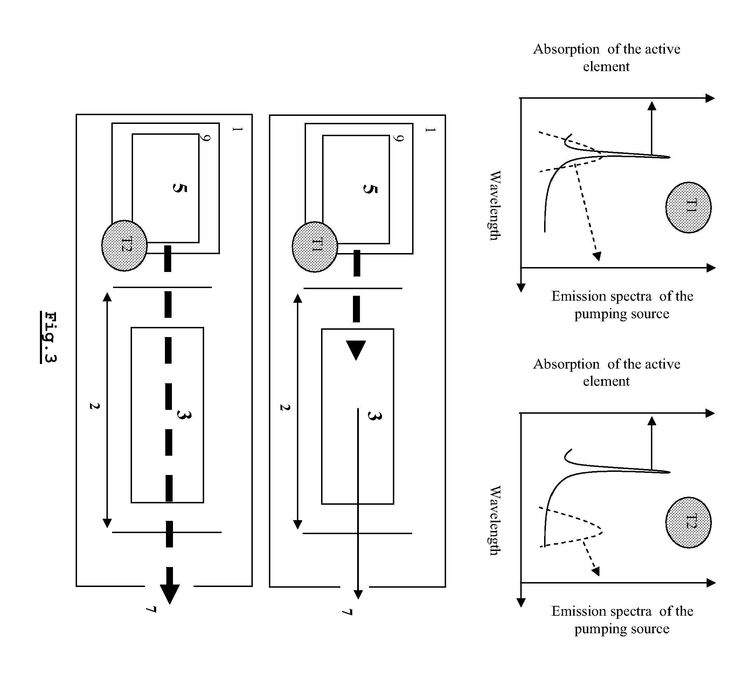

The invention is further explained for the case of fibre lasers. In a fibre laser or amplifier, the pumping laser diode wavelength is generally selected to match an absorption peak of the doped fibre spectra. Then the pumping signal is absorbed by the fibre and re-emitted at a different wavelength depending on the nature of the doping elements into the fibre (active element). This is shown in Fig.3 by the illustrations at T1. T1 denotes the pumping source temperature at which the pumping source emission matches the absorption peak of the active element (3) of the laser device (1).
In case high peak absorption is present in the active element (3), the pump (5) can achieve a high absorption. This however leads to a device that is less stable and more sensitive to the pumping wavelength fluctuations. By slightly detuning the pumping diode wavelength, it is then possible to move the pumping signal away from the absorption peak of the active element (3), such that the above-mentioned drawbacks are mitigated. This is performed in most cases in order to stabilize the fibre laser operation and to avoid problems due to external temperature changes for instance. Generally this principle of slightly detuning is applied for telecom applications.

For achieving said tuneability the laser device (1) may typically be provided with a thermo-electric control element (9). This may be a high thermal capacity thermo-electric management system. The amount of wavelength shift needed to achieve the desired effect of operating the laser device with other characteristics depends on the type of active element used. For a co-doped Erbium-Ytterbium fibre for example, a shift over 10 nm is suitable. A typical wavelength shift of pump diodes with temperature is 0.3nm/°C. The cooling system then needs to be designed in order to allow the pumping laser diode to change in temperature over 30 to 40°C in order to obtain a wavelength shift of about 10 nm, which would lead to a change of mode of operation of the device.

However, by detuning the pumping wavelength completely out of the peak spectra it is possible to substantially emit this pumping light instead of active element's generated signal. Indeed, the pumping light is in that case almost no longer absorbed by the active element and constitutes the main part of the emission at the laser device output (7), while the signal generated by the active element is considerably decreased. Fig.3 further shows an illustration at temperature T2 at which the emission peak of the pumping source (5) is sufficiently shifted away of the absorption spectra in order to be the predominant emitted signal. The output signal of the fibre laser or amplifier for example can then be switched between the pumping signal and the light emission of the doped fibre (which is the active element). This allows switching between two wavelength emission regions for instance. This feature is particularly interesting for medical applications for instance where, due to the spectra of absorption of the skin, the feature of having a source emitting for example around 980nm or 1550nm allows addressing different problems/applications with the same device.

A most preferred embodiment of the invention relates to a double clad doped fibre laser, wherein the doped fibre is the active element of the laser and a multi-mode laser diode is the pumping source.
With such a double clad fibre system it is also possible to tune in the way set out above the spatial shape of the output signal. In double clad fibre lasers or fibre amplifiers, the pumping diodes are multi-mode, whereas the emitted signal at the output is single-mode or nearly single-mode. The present invention allows switching between the multi-mode operation of the pumping signal and the single-mode operation of the fibre laser. Then one single laser device can have different beam shapes depending on the applied switching principle. The device can be an interesting adaptive tool for material processing for instance.

In order to obtain a high performance, an appropriate doped fibre absorption and length have to be selected. When the pumping wavelength matches the absorption peak spectra of the doped fibre, most of the output radiation (generally >80% or >90%) consists of the light generated by the processes of absorption and emission of the pump in the active element (doped fibre). Nevertheless a small amount of pump radiation remains as in most of the fibre lasers devices. This residual pump emission (which is at very low power as compared to signal emission) turns out to become predominant when its wavelength is tuned. When sufficiently tuned away of the doped fibre absorption peak, the device output mainly (>80%) consists of the emission of the pumping signal. To achieve such result, the doped fibre length has to be optimized for the two modes of operation. The addition of a long period grating (LPG) (10) can significantly improve the performance of the device, as it increases the coupling between the pump core and the signal core (in the case of double clad fibres) thereby enhancing the absorption of the pump at a given wavelength. The use of a pump reflector (11) at the output of the doped fibre also enhances the switching between the two modes of operation. It allows improving the contrast between pump and signal radiation. The doped fibre lengths can be reduced and another pumping wavelength selective element can be added to the device to render it more sensitive to pumping wavelength shifts. Note that in the case of double-clad devices this pump reflector should be a fibre Bragg grating (FBG) inscribed onto a highly multimode guiding fibre.

The method can also be significantly improved by providing the laser device (e.g. a fibre laser) at its input with a wavelength selective or reflective element (for instance a dichroic mirror) that closely matches the pumping wavelength. Fig.4 illustrates this embodiment. This wavelength selective element (6) allows increasing the switching ratio between two modes of operation. Dichroic mirrors (6) can also be used in fibre lasers as end face elements that allow reflection of the fibre laser wavelength radiation and transmission of the pumping source. As shown in Fig.4, the dichroic mirror (6) preferably has a very sharp spectral transmission curve close to the pump wavelength. For instance a sharp bandpass, short-pass of long-pass dichroic mirror can be employed. The mirror transmits (or reflects, respectively) the pump radiation to the doped fibre when its wavelength corresponds to the peak absorption spectra and then reflects (or transmits) it when a significant wavelength shift is applied. This improvement yields a better contrast between the two modes of operation of the device. However it calls for two different fibre outputs (7,8) unless a multiplexer is used to recombine both signals. This is illustrated in Fig.4. Such improvement also allows decreasing the required wavelength shift between the two modes of operation.
In the case of a double clad fibre laser, the best solution consists in providing a dichroic mirror with very precise and well chosen characteristics. Then a change of the pump wavelength allows a shift from a transmission to reflection state on the dichroic mirror. The necessary wavelength shift of the pumping source depends on the sharpness of the dichroic mirror transmission curve. No mirror rotation or other mechanical action is necessary to change the mode of operation of the device. In that way a mechanically stable system is achieved.

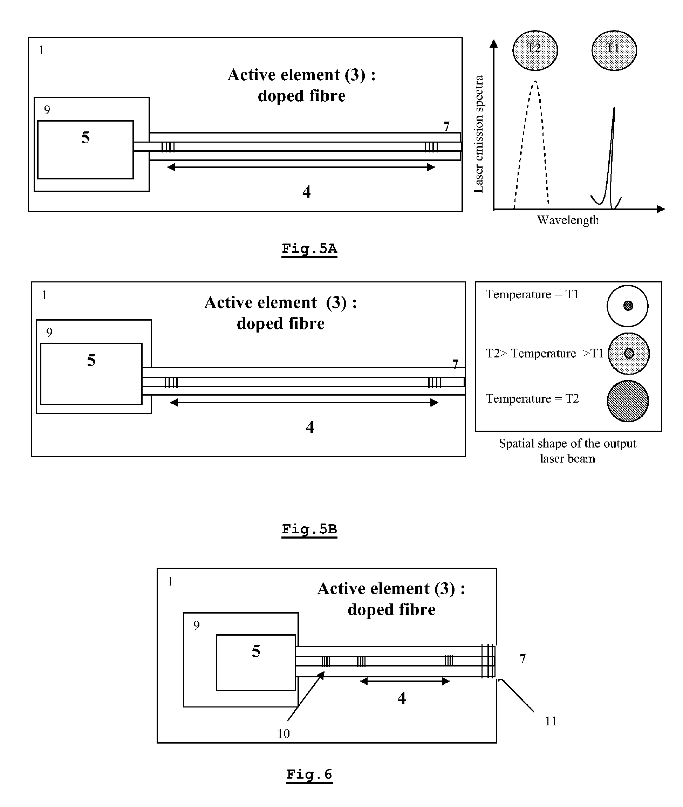

Fig.5 shows some more possible embodiments.
Fig.5A relates to wavelength tuning of a fibre laser (e.g. based on an Erbium doped fibre or a co-doped Erbium-Ytterbium fibre, but also other doping elements are usable). The pumping diode has an emission wavelength of 976nm, corresponding to a maximum peak absorption of Erbium. By tuning for instance the diode temperature from T1 to T2, it is possible to shift its wavelength away from the absorption maximum of the fibre. Then the pump signal propagates through the whole fibre laser structure without being absorbed or only minimally absorbed and is emitted instead of the 1.55µm original signal. In this way a double wavelength emitting laser device is obtained. This laser is operable in two very different wavelength regions: around 1µm and around 1.55µm. It can be applied in very different applications as surgery and dermatology in the case of medical applications. Indeed the 1.55µm wavelength matches an absorption peak of water, whereas the 1µm region is well absorbed by melanin, these two elements both being present in the human skin.

In Fig.5B beam shape tuning is illustrated for the case of a double clad doped fibre. A double clad Ytterbium doped fibre is considered, again pumped by a 976nm laser diode. When no tuning is applied to the pumping diode, the radiation is absorbed by the Ytterbium doping element in the fibre. Next, the radiation is converted into a 1µm single-mode signal, as the Ytterbium is mainly present in the single-mode core of the double clad fibre. However, when the pumping radiation wavelength is tuned by shifting to temperature T2, it is no longer absorbed by the doped core of the fibre. It can then propagate through the multi-mode core of the double clad fibre. Consequently, the laser device output is tuned from a single-mode operation to a multi-mode operation. In this example again a spectral shift is obtained, but, more importantly, the device is given a new feature in that it is capable of switching between two spatial beam qualities. This is of particular interest in the case of material processing as for instance in laser marking.

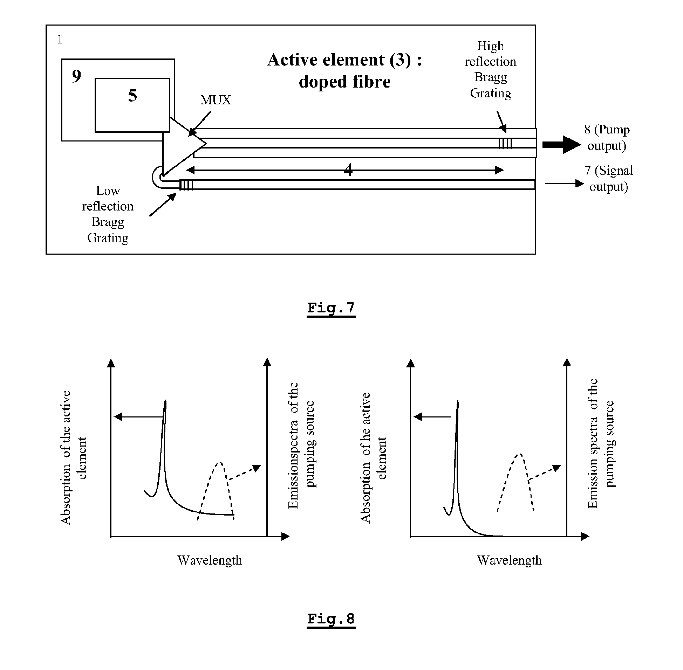

Fig.6 shows an improvement of the design in order to have a better extinction ratio (i.e. the ratio of emitted light from the second characteristics to emitted light from the first one) between the two wavelengths. In this configuration the device has two different outputs. The pump radiation output and the active element generated light output are at opposite ends of the laser cavity. This allows obtaining a better contrast between the pump and generated light for the two modes of operation.

Fig.7 shows an embodiment wherein wavelength depending elements are added to improve the pump absorption at the wavelength corresponding to the peak absorption spectra of the active element (3). This allows reducing the length of the active element. As a consequence, when a large detuning is applied to the pump source (5), the residual absorption in the switched mode is avoided.

Fig.8 shows a further embodiment of the invention by working on the active element in order to limit the absorption coefficient away from the absorption peak. The invention can be improved by optimising the absorption spectra of the gain medium, for instance a sharper absorption peak of the doped fibre can help to have a switch with a lower required tuning range for the pump. Also an optimized pump system, with high wavelength dependency with temperature or combining Bragg grating and thermal effect, constitutes an interesting embodiment of the invention.

Other possible improvements of the invention can be envisaged. Again, as already explained, the principle of invention is to switch laser or amplifier properties by tuning its pumping wavelength out of the absorption peak of the active element absorption band. This is applicable to fibre based lasers and amplifier but also to solid-state, dye or gas ... pumped by another optical source. As well an optimised pump system, with high wavelength dependency with temperature or combining Bragg grating and thermal effect could also be an improvement of this invention.

## Claims

1. Laser device (1) comprising a double clad active element (3) for emitting light and a multi-mode pumping source (5) arranged for providing energy to said double clad active element (3), said laser device being arranged for emitting light at a first wavelength determined by an absorption spectrum of said double clad active element and whereby the laser device is further arranged for emitting light at a second wavelength determined by said multi-mode pumping source (5), said multi-mode pumping source (5) being tuneable away from said absorption spectrum.

2. Laser device as in claim 1, wherein said tuneable multi-mode pumping source (5) is a laser diode.

3. Laser device as in claim 1 or 2, wherein said laser device is a double clad fibre laser or a fibre amplifier.

4. Laser device as in any of claims 1 to 3, further comprising a long period grating.

5. Laser device as in any of claims 1 to 4, further comprising a pump reflector at an output (7) of said laser device.

6. Laser device as in any of the previous claims, further comprising a wavelength selective or reflective element.

7. Laser device as in claim 6, whereby said wavelength selective or reflective element is a dichroic mirror.

8. Laser device as in any of the previous claims, wherein said second wavelength is the wavelength generated by said multi-mode tuneable pumping source.

9. Medical device comprising a laser device as in any of the previous claims.

10. Method for switching between operable states of a laser device comprising the steps of :
- providing a laser device (1) comprising a double clad active element (3) and a tuneable multi-mode pumping source (5),
- emitting with said laser device (1) light at a first wavelength determined by an absorption spectrum of said double clad active element (3),
- tuning said tuneable pumping source (5), such that a wavelength shift to a second wavelength away from said absorption spectrum is obtained,
- emitting with said laser device (1) light at said second wavelength.

11. Method for switching as in claim 10, whereby light is first emitted at said second wavelength, whereby subsequently said tuneable pumping source (5) is tuned to said absorption spectrum and whereby then light is emitted at said first wavelength.

12. Method for switching as in claim 10 or 11, wherein said step of tuning is performed by changing the temperature of said tuneable pumping source (5).

13. Method for switching as in any of claims 10 to 12, wherein said laser device further comprises a wavelength dependent element, such that said step of tuning can be performed over a smaller range.

14. Method for operating a medical device, comprising the step of carrying out the method as in any of claims 10 to 13.
